# EUROPÄISCHE PATENTSCHRIFT

(11) **EP 1 687 040 B1**
(45) Veröffentlichungstag und Bekanntmachung des Hinweises auf die Patenterteilung: **20.02.2013**
(21) Anmeldenummer: 04798078.4
(22) Anmeldetag: 25.11.2004
(51) Int. Cl.: A61L 24/00, A61L 27/42, A61L 27/46, A61L 27/56, A61L 27/58

(54) **BIORESORBIERBARES KOMPOSITMATERIAL**
BIORESORBABLE COMPOSITE MATERIAL
MATERIAU COMPOSITE BIORESORBABLE

(30) Priorität: 27.11.2003 DE 10355992
(43) Veröffentlichungstag der Anmeldung: 09.08.2006
(73) Patentinhaber: Curasan AG, 65933 Frankfurt/Main (DE)
(72) Erfinder: SCHNABELRAUCH, Matthias, 07749 Jena (DE); VOGT, Sebastian, 99084 Erfurt (DE); REIF, Dieter, 98553 Altendambach (DE)
(74) Vertreter: Forstmeyer, Dietmar
(86) Internationale Anmeldenummer: PCT/EP2004/013388
(87) Internationale Veröffentlichungsnummer: WO 2005/051443

(56) Entgegenhaltungen:
- WO-A-87/00058
- DE-A1- 19 939 403
- US-A- 4 192 021
- US-A1- 2002 120 033

## Beschreibung

Die vorliegende Erfindung betrifft ein Verfahren zur Herstellung eines selbsthärtenden, bioresorbierbaren Kompositmaterials, ein selbsthärtendes, bioresorbierbares Kompositmaterial und erlaubt dessen Verwendung in der Human- und Veterinärmedizin, insbesondere zur Verklebung von Knochengewebe, zur Auffüllung von Knochendefekten und zur Herstellung von implantierbaren Formkörpern.

Nicht oder nur teilresorbierbare Knochenzemente sind seit langem bekannt und wurden in einer Vielzahl von Patenten und wissenschaftlichen Publikationen beschrieben (z.B. G. Lewis, J. Biomed. Mater. Res. (Appl. Biomater.) 38 (1997) 155). Sie bestehen im allgemeinen aus einer flüssigen und einer festen Komponente. Die flüssige Komponente besteht aus einem flüssigen monofunktionellen Methacrylsäureester, wobei das Methylmethacrylat bevorzugt verwendet wird. In diesem Monomer ist ein Polymerisationsaktivator, im allgemeinen N,N-Dimethyl-p-toluidin, gelöst. Die feste Komponente besteht aus einem in dem Monomer quellbaren bzw. löslichen Polymer, wobei Copolymere des Methylmethacrylates und des Methylacrylates am häufigsten eingesetzt werden. In der festen Komponente ist ein Polymerisationsinitiator, wie Dibenzoylperoxid, enthalten. Die Aushärtung dieser Knochenzemente erfolgt in der Weise, daß nach dem Vermischen der festen mit der flüssigen Komponente, der im Monomer gelöste Polymerisationsaktivator auf den in der festen Komponente enthaltenen Polymerisationsinitiator trifft. Durch Einwirkung des Polymerisationsaktivators zerfällt der Polymerisationsinitiator unmittelbar unter Entstehung von Radikalen, die sofort die Polymerisation des monofunktionellen Monomers auslösen. Es entstehen durch Polymerisation der monofunktionellen Monomere nicht vernetzte Polymere, die im noch nicht umgesetzten Monomer löslich beziehungsweise quellbar sind. Dadurch bleibt der Zement über einen Zeitraum von mehreren Minuten plastisch verformbar und kann verarbeitet werden. Die Polymerisationsgeschwindigkeit ist nur abhängig von der Initiator- und der Monomerkonzentration und verläuft daher relativ langsam.

Mit mehrfachfunktionellen Monomeren sind diese Knochenzemente nicht mehr handhabbar, weil mehrfachfunktionelle Monomere auf Grund ihrer Polymerisationskinetik extrem schnell polymerisieren und zu festen Polymernetzwerken führen, die auch schon bei geringen Vernetzungsgraden nicht mehr plastisch verformt und damit verarbeitet werden können.

Im US 5814682 wird eine Komposition beschrieben, die aus einer Paste A, bestehend aus einem Gemisch eines polymerisierbaren Monomers, eines Initiators und Calciumphosphat, und einer Paste B besteht, die aus einem Gemisch eines polymerisierbaren Monomers, eines Aktivators und Calciumphosphat gebildet wird.

Nach Vermischung der Pasten A und B polymerisiert die Komposition.

Ein dem US 5814682 ähnliches Kompositsystem wird im WO 87/00058 beschrieben. Ein Knochenzement auf Basis Diacrylat oder Dimethacrylat enthält resorbierbare Partikel aus Biokeramik oder Bioglas. Die anorganischen Füllstoffpartikel sollen ein Porenvolumen von mindestens 0,2 ml/g aufweisen, da sich dies günstig auf die physikalischen Eigenschaften des Zementes auswirken soll.

Ein radikalisch polymerisierbarer Dentalwerkstoff wurde im EP 0951896 A2 beschrieben. Dieser Dentalwerkstoff ist dadurch gekennzeichnet, daß der Füllstoff eine homogene Mischung aus einem ersten Teil des Füllstoffes, der mit dem Polymerisationsinitiator beschichtet ist, einem zweiten Teil des Füllstoffes, der mit dem Polymerisationsaktivator beschichtet ist, und einem dritten Teil des Füllstoffes darstellt, der keine Komponenten des Initiatior-Systems enthält.

Im DE 19939403 A1 ist ein biologisch abbaubares Kompositmaterial offengelegt. Das Kompositmaterial entsteht durch Aushärten aus der Mischung einer flüssigen Komponente A, die mindestens ein polymerisierbares, bioresorbierbares Monomer sowie gegebenenfalls ein bioresorbierbares Verdickungsmittel enthält, einer festen Komponente B, die aus einem mit einem Polymerisationsinitiator beschichteten, bioresorbierbaren anorganischen Füllstoff besteht sowie einer festen Komponente C, die aus einem mit einem Polymerisationsaktivator beschichteten, bioresorbierbaren anorganischen Füllstoff gebildet wird. Bei diesem Kompositsystem sind der Polymerisationsaktivator und der Polymerisationsinitiator auf der Oberfläche von Füllstoffen als Beschichtung aufgebracht. Bei dem anorganischen Füllstoff kann es sich um Calciumcarbonat, Magnesiumcarbonat; Calciumphosphat und . Hydroxylaptit handeln, ohne dass interkonnektierende Porösität gefordert wird.

DE 198 18 210 A1 beschreibt einen radikalisch polymerisierbaren Dentalwerkstoff mit mindestens einem polymerisierbaren Bindemittel und mindestens einem Füllstoff, enthaltend ein Redox-Initiator-System für die radikalische Polymerisation, wobei das System einen Initiator und einen Aktivator umfaßt. Bei dem bekannten Wirkstoff ist der Füllstoff eine homogene Mischung aus einem ersten Teil des Füllstoffes, der mit dem Initiator gemischt ist, einem zweiten Teil des Füllstoffes, der mit dem Aktivator gemischt ist, und einen dritten Teil des Füllstoffes, der keine Komponente des Initiator-Systems enthält. Für den Füllstoff wird wiederum keine interkonnektierende Porösität gefordert, wobei als bevorzugte Füllstoffe beispielsweise Quarzpulver, Glaskeramikpulver, Glaspulver, Aluminiumpulver und Siliziumoxidpulver angesprochen werden.

US 5,814,681 betrifft eine restaurative Komposition für Hartgewebe mit anorganischem Calciumphosphat-Pulver, wobei wiederum keine interkonnektierende Porösität gefordert wird. Als polymerisierbare Monomere werden ω,ω-ungesättigte Verbindungen vorgesehen, für deren Polymerisation folgende Warnung ausgesprochen wird:
"Excess time for mixing lead to the initiation of cooling before application of the mixture to the affected part, thereby making the mixture unavailable."

DE 44 35 680 A1 betrifft die Herstellung eines porösen Knochenersatzmaterials unter Verwendung eines anorganischen Ausgangsmaterials, für das keine Porösität vorgeschrieben wird. Zur Herstellung des bekannten Knochenersatzmaterials wird von einem Gemenge (a) eines Polymerisats mit Polymerisationskatalysator, (b) eines flüssigen Monomeren mit Polymerisationsbeschleuniger und (c) anorganischem Material in Form von grobteiligem Granulat ausgegangen.

DE 100 18 394 A1 betrifft die Herstellung von porösen Calciumphosphosphat-Körpern, die durch Sintern erhalten werden.

Der Stand der Technik selbsthärtender, bioresorbierbarer Kompositmaterialien wird demnach dadurch charakterisiert, dass die für eine Vernetzung der Monomere erforderlichen Polymerisationsaktivatoren und Polymerisationsinitiatoren den unterschiedlichen Komponenten des Kompositsystems untergemischt oder auf der Oberfläche dieser Komponenten aufgebracht werden. Der vorliegenden Erfindung liegt nun die Aufgabe zugrunde, die Reaktion dieser Beschleunigerkomponenten bei mehrfach-funktionellen Monomeren noch besser steuern zu können.

Ferner liegt der Erfindung die Aufgabe zu Grunde, ein neues, selbsthärtendes, bioresorbierbares Kompositmaterial auf der Grundlage di-, tri- oder anderer mehrfach-funktioneller Monomere als Polymernetzwerkbildner zu schaffen. Dieses Kompositmaterial soll durch Vermischen einzelner Kompositbestandteile und/oder vorgefertigter Teilmischungen oder Teilreaktionsprodukten zu einer gieß-, spritz- oder streichfähigen Masse herstellbar sein und bei Raumtemperatur nach einer Verarbeitungszeit von 2 bis 5 Minuten zu einem festen Verbund selbständig aushärten.

Die der Erfindung zugrundeliegende Aufgabe wird nun gemäß einer Ausführungsform durch ein Verfahren zur Herstellung eines selbsthärtenden bioresorbierbaren Kompositmaterials gelöst, bei dem man
(i) einen Polymerisationsinitiator mit Hilfe einer ersten Teilmenge eines interkonnektierend-porösen bioresorbierbaren anorganischen Knochenregenerationsmaterials immobilisiert,
(ii) einen Polymerisationsaktivator mit Hilfe einer zweiten Teilmenge des Knochenregenerationsmaterials gemäß (i) oder eines anderen interkonnektierend-porösen bioresorbierbaren anorganischen Knochenregenerationsmaterials immobilisiert,
(iii)die bei den Stufen (i) und (ii) anfallenden Komponenten mit einem flüssigen oder pastösen zu einem biokompatiblen und bioresorbierbaren Polymer polymerisierbaren mehrfunktionellen Monomer oder mit einer flüssigen oder pastösen Mischung zu einem biokompatiblen und bioresorbierbaren Polymer polymerisierbaren mehrfunktioneller Monomerer mischt und
(iv) das mit der erhaltenen Mischung vermischte Monomer oder Monomergemisch zu dem Polymer polymerisiert und das Kompositmeterial gewinnt.

Die Aufgabe wird also erfindungsgemäß durch die Schaffung eines neuen Verfahrens zur Herstellung eines selbsthärtenden, bioresorbierbaren Kompositmaterials gelöst, das drei wesentliche Verfahrensschritten umfaßt. Dabei wurde überraschend gefunden, dass durch poröse Calciumphosphate mit interkonnektierendem Porensystem bzw. einem interkonnektierendporösem System, beispielsweise einem mikroporösen Porensystem, Polymerisationsinitiatoren und Polymerisationsaktivatoren so immobilisiert werden können, dass diese durch flüssige oder pastöse Monomere nicht spontan, sondern über einen verlängerten Zeitraum aus den Porensystemen eluiert werden.

Dieser Immobilisierungseffekt läßt sich beispielsweise dann noch steigern, wenn man Initiator und Aktivator, nachdem sie mit dem teilchenförmigen, partikulären bzw. gekörnten porösen Calciumphosphat immobilisiert worden sind, von den Ober- bzw. Außenflächen der Teilchen wieder ablöst und nur in Poren verweilen läßt, bevor man das Monomere zusetzt.

Eine derartige partielle Ablösung läßt sich beispielsweise dadurch fördern, dass man nur kurz mit einem Lösungsmittel benetzt und/oder das Lösungsmittel nicht in die Poren eindringen läßt, indem man beispielsweise die Poren nicht evakuiert.

Bei dem erfindungsgemäßen Verfahren kann man Bestandteile zumischen, insbesondere bei Stufe (iii), die die Eigenschaften des Monomer, des Monomergemischs und/oder des Kompositmaterials modifizieren.

So kann man bei dem erfindungsgemäßen Verfahren Bestandteile zumischen, insbesondere bei Stufe (iii), die die Eigenschaften des Monomers, des Monomergemischs und/oder des Kompositmaterials modifizieren. Solche Bestandteile können beispielsweise Substanzen sein, die die Viskosität des Monomers, Monomergemischs und/oder deren Mischung mit dem Knochenregenerationsmaterial in einer für die Applikation wünschenswerten Weise verändern. Weitere solche zumischbaren Bestandteile können den pH-Wert verändernde Substanzen, Porenbildner (sogenannte Porogene), Haftvermittler, Farbstoffe, Kontrastmittel und/oder pharmazeutische Wirkstoffe sein.

So kann man einen oder mehrere modifizierende Bestandteile zumischen, die aus der Gruppe ausgewählt sind von: Verdickungsmittel, Verdünnungsmittel, polymerer Füllstoff, Porogen, pH-modifizierende Substanz, Farbstoff und Adhäsionsvermittler.

Ferner kann man bei dem erfindungsgemäßen Verfahren die erste Teilmenge und die zweite Teilmenge des Knochenregenerations-materials in einem Verhältnis von 1:10 bis 10:1 einsetzen und/oder den Polymerisationsinitiator und den Polymerisationsaktivator mit bzw. in den jeweiligen Teilmengen des Knochenregenerationsmaterials in einem Verhältnis von 1:10 bis 10:1 immobilisieren (jeweils auf Massebasis).

Ferner kann man bei dem erfindungsgemäßen Verfahren das Knochenregenerationsmaterial in Form von Pulver oder Granulat einsetzen.

Ferner kann man bei dem erfindungsgemäßen Verfahren bei Stufe (i) das Knochenregenerationsmaterial mit einer Lösung des Polymerisationsinitiators versetzen, die Lösung das Knochenregenerationsmaterial infiltrieren lassen und danach das Knochenregenerationsmaterial trocknen.

Ferner kann man bei dem erfindungsgemäßen Verfahren eine Lösung des Polymerisationsinitiators in einer Menge von 0,1 bis 20 Masse-% mit der zu seiner Immobilisierung vorgesehenen Menge (Teilmenge) an Knochenregenerationsmaterial vermischen (fester Initiator bezogen auf Knochenregenerationsmaterial).

Ferner kann man bei dem erfindungsgemäßen Verfahren ein organisches Peroxid als Polymerisationsinitiator verwenden, vorzugsweise ein aus der Dibenzoylperoxid, Lauroylperoxid und Acetonperoxid umfassenden Gruppe ausgewähltes organisches Peroxid.

Ferner kann man bei dem erfindungsgemäßen Verfahren bei Stufe (ii) das Knochenregenerationsmaterial mit einer Schmelze oder einer Lösung des Polymerisationsaktivators versetzen, die Lösung das Knochenregenerationsmaterials infiltrieren lassen und danach das Knochenregenerationsmaterial trocknen.

Ferner kann man bei dem erfindungsgemäßen Verfahren eine Lösung des Polymerisationsaktivators in einer Menge von 0,1 bis 20 Masse-% mit der zu seiner Immobilisierung vorgesehenen Menge (Teilmenge) an Knochenregenerationsmaterial vermischen (fester Aktivator bezogen auf Knochen-regenerationsmaterial).

Ferner kann man bei dem erfindungsgemäßen Verfahren einen oder mehrere Polymerisationsaktivatoren verwenden, die aus der N,N-Bis-(2-hydroxyethyl)-p-toluidin, N,N-Dimethyl-p-toluidin, N,N-Dimethyl-N,N-anilin, Ascorbinsäure und Barbitursäure umfassenden Gruppe ausgewählt sind.

Ferner kann man bei dem erfindungsgemäßen Verfahren den Polymerisationsinitiator in Form einer Lösung und/oder den Polymerisationsaktivator in Form einer Lösung einsetzen und die Lösung (en) vom Knochenregenerationsmaterial vollständig aufsaugen lassen oder soweit wie möglich aufsaugen lassen und den nicht aufgesaugten Überschuß vor Stufe (iii) entfernen.

Ferner kann man bei dem erfindungsgemäßen Verfahren ein Erdalkaliphosphat und/oder ein Alkali/Erdalkaliphosphat als anorganisches Knochenregenerationsmaterial verwenden, insbesondere ein Erdalkali-orthophosphat und/oder Alkali/Erdalkali-orthophosphat, vorzugsweise ein Knochenregenerationsmaterial, das aus der alpha-Tricalciumphosphat, beta-Tricalciumphosphat, calciumdefizitären carbonathaltigen Hydroxylapatit, Octacalciumphosphat, Magnesiumphosphat, Calciumhydrogenphosphat, Calcium/Natriumorthophosphat und Calciumpyrophosphat umfassenden Gruppe ausgewählt ist.

Ferner kann man bei dem erfindungsgemäßen Verfahren für die Immobilisierung des Polymerisationsinitiators dasselbe oder ein anderes Knochenregenerationsmaterial verwenden als für die Immobilisierung des Polymerisationsaktivators. Diese freie Wahl entspricht insoweit dem Stand der Technik etwa gemäß US 5 814 681 Spalte 3 Zeilen 53-55.

Dabei kann sich das Knochenregenerationsmateral für die Immobilisierung des Initiators von dem Knochenregenerationsmateral für die Immobilisierung des Aktivators durch seine chemische und/oder mineralogische Natur unterscheiden.

Ferner kann man bei dem erfindungsgemäßen Verfahren ein interkonnektierend-poröses Knochenregenerationsmaterial, insbesondere Calciumphosphat, mit folgenden Kenndaten verwenden:
- Porendurchmesser von 0,1 bis 500 *µ*m, vorzugsweise von 0,1 bis 100 *µ*m, und/oder
- Korngrößen (d₅₀-Werte) von 1 bis 500 *µ*m, vorzugsweise 5 bis 300 *µ*m, und/oder
- Oberfläche nach BET von wenigstens 0,1 m²/g.

Ferner kann man bei dem erfindungsgemäßen Verfahren ein interkonnektierend-poröses Knochenregenerationsmaterial, insbesondere Calciumphosphat, mit einem für den Polymerisationsinitiator und/oder den Polymerisationsaktivator zugänglichen Porenvolumen von 0,4 cm³/g oder mehr bei Erhalt der Integrität der Teilchen des Knochenregenerationsmaterials und insbesondere von 0,4 bis 3,3 cm³/g einsetzen.

Ferner kann man bei dem erfindungsgemäßen Verfahren das Knochenregenerationsmaterial, insbesondere Calciumphosphat, in kristalliner, teilkristalliner, glasiger oder amorpher Form verwenden.

Ferner kann man bei dem erfindungsgemäßen Verfahren dem Knochenregenerationsmaterial Bestandteile zumischen, die biokompatibel sind und die Eigenschaften des Regenerationsmaterials modifizieren, insbesondere Siliziumverbindungen.

Ferner kann man bei dem erfindungsgemäßen Verfahren als Monomer oder als Monomere des Monomergemischs ein mehrfunktionelles Oligomeres mit endständigen Methacrylatgruppen verwenden, insbesondere ein Oligomeres von Milchsäure und/oder Glykolsäure und/oder delta-Hydroxyvaleriansäure und/oder epsilon-Hydroxycapronsäure und/oder Trimethylencarbonat.

Dabei kann man das Monomer oder das Monomergemisch zusammen mit einem Haftvermittler verwenden, vorzugsweise einem hydroxylgruppenhaltigen Haftvermittler, insbesondere Methacrylsäure-2-hydroxyethylester.

Ferner kann man bei dem erfindungsgemäßen Verfahren das Monomer oder das Monomergemisch zusammen mit einer viskositätsmodifizierende Substanz bzw. einem Verdicker verwenden, vorzugsweise Dianhydro-D-glucit-bis-(poly-D,L-lactid).

Gemäß einer weiteren Ausführungsform wird die der Erfindung zugrundeliegende Aufgabe durch ein selbsthärtendes bioresorbierbares Kompositmaterial als Set gelöst, bestehend aus oder umfassend
(i) eine erste Teilmenge eines interkonnektierend-porösen bioresorbierbaren anorganischen Knochenregenerationsmaterials und einen Polymerisationsinitiator, der mit Hilfe dieser ersten Teilmenge immobilisiert ist,
(ii) eine zweite Teilmenge des Knochenregenerationsmaterials gemäß (i) oder eines anderen interkonnektierend-porösen bioresorbierbaren anorganischen Knochenregenerations-materials und einen Polymerisationsaktivator, der mit Hilfe dieser zweiten Teilmenge immobilisiert ist, und
(iii)ein flüssiges oder pastöses zu einem biokompatiblen und bioresorbierbaren Polymer polymerisierbares mehrfunktionelles Monomer oder eine flüssige oder pastöse Mischung zu einem biokompatiblen und bioresorbierbaren Polymer polymerisierbarer mehrfunktioneller Monomerer.

Bei dem erfindungsgemäßen Kompositmaterial kann das Massenverhältnis von Knochenregenerationsmaterial : Monomer oder Monomergemisch 4 : 6 bis 7 : 3 betragen..

Das erfindungsgemäße Kompositmaterial kann als Set nach dem erfindungsgemäßen Verfahren erhältlich sein, so wie jede seiner Komponenten (i), (ii) und (iii).

Schließlich wird die der Erfindung zugrundeliegende Aufgabe dadurch gelöst, dass man ein selbsthärtende Knochen-regenerationsmaterial in Form eines erfindungsgemäßen Sets zur Herstellung eines Knochenklebers für die Fixierung von Knochenfrakturen verwendet.

Erfindungsgemäß wird also in einem ersten Teilschritt (I) ein Polymerisationsinitiator in einem interkonnektierenden Porensystem einer ersten Teilmenge eines zur Herstellung des selbsthärtenden, bioresorbierbaren Kompositmaterials eingesetzten, bioresorbierbaren Knochenregenerationsmaterial, z.B. eines Calciumphosphates immobilisiert. In einem weiteren Verfahrensschritt (II) wird in dem interkonnektierenden Porensystem einer zweiten Teilmenge des zur Herstellung des selbsthärtenden, bioresorbierbaren Kompositmaterials verwendeten bioresorbierbaren Knochenregenerationsmaterials, z.B. eines Calciumphosphates ein Polymerisationsaktivator immobilisiert. Die erste und die zweite Teilmenge verhalten sich dabei wie 1:10 bis 10:1 und der Polymerisationsinitiator und der Polymerisationsaktivator verhalten sich dabei wie 1:10 bis 10:1 (jeweils auf Massebasis). Die Komponenten nach (I) und (II) werden in einem dritten Verfahrensschritt (III) homogen mit einem zur Bildung eines biokompatiblen, bioresorbierbaren Polymernetzwerkes befähigten, flüssigen oder pastösen Monomer oder einer Monomermischung und gegebenenfalls, weiteren, die Eigenschaften des Monomers modifizierenden Bestandteilen vermischt. Solche eigenschaftsmodifizierenden Bestandteile können z. B. Substanzen sein, die die Viskosität des Monomers, Monomergemischs und/oder deren Mischung mit dem Knochenregenerationsmaterial in einer für die Applikation wünschenswerten Weise verändern. Weitere solche zumischbaren Bestandteile können den pH-Wert verändernde Substanzen, Porenbildner (sogenannte Porogene), Haftvermittler, Farbstoffe, Kontrastmittel und/oder pharmazeutische Wirkstoffe sein. Für die Menge an Monomer oder Monomermischung sowie an modifizierenden Bestandteilen kann auf den Stand der Technik verwiesen werden.

Unter Immobilisierung wird im Rahmen der vorliegenden Erfindung vorzugsweise das temporäre Fixieren einer reaktionsbeschleunigenden Substanz in dem interkonnektierenden Poren- oder Kanalsystem geeigneter, im wesentlichen nicht selbst reagierender Trägerpartikel aus Knochenregenerationsmaterial, z.B. Calciumphosphat verstanden. Zur Größe der Trägerpartikel kann auf den Stand der Technik verwiesen werden. Grundsätzlich kann man sich auch eine Anwendung weiterer, aus Drug Delivery Systemen bekannter Prinzipien mit verzögerter Wirkstofffreisetzung in Rahmen des neuen Verfahrens vorstellen; vgl. beispielsweise Schmidt et al. in J. Controlled Release, 37 (1995) 83-94 und Cimbollek et al. in Antimicrob. Agents Chemother., 40 (1996) 1432-1437. Entscheidend für eine Anwendbarkeit ist die passende Freisetzungsgeschwindigkeit für die Lösung der erfindungsgemäßen Aufgabe.

Werden derartige interkonnektierend-poröse Knochenregenerationsmaterialien, z.B. Calciumphosphate, in denen mindestens ein Polymerisationsinitator immobilisiert ist, und interkonnektierend-poröse Knochenregenerationsmaterialien, z.B. Calciumphosphate, in denen mindestens ein Polymerisationsaktivator immobilisiert ist, mit flüssigen oder pastösen mehrfach-funktionellen polymernetzwerkbildenden Monomeren oder Monomermischungen vermischt, so lassen sich diese Gemische bei Raumtemperatur überraschend in einem Zeitraum von 2-10 Minuten verarbeiten. In dieser Zeit sind die Gemische plastisch verformbar und spritz-, gieß- und streichfähig. Danach setzt die Aushärtung schlagartig ein.

Dieser Befund ist auf Grund der an sich bekannten Polymerisationskinetik von mehrfach-funktionellen Monomeren überraschend. Bei der bekannten Polymerisationskinetik würde man eine Verarbeitungszeit von wenigen Sekunden erwarten.

Man kann annehmen, dass die Verarbeitungszeit des selbsthärtenden, bioresorbierbaren Kompositmaterials durch verzögerte Diffusion aus den Porensystemen der porösen Knochenregenerationsmaterialien wie z.B. Calciumphosphate bestimmt wird. Ferner wird angenommen, dass der Poren- und Kanaldurchmesser des interkonnektierenden Porensystems und die Größe der interkonnektierenden Porensysteme Einfluss auf die Diffusion haben, bestimmt durch die Partikelgrößenverteilung der bioresorbierbaren Knochenregenerationsmaterialien wie z.B. Calciumphosphate. Daneben wird die Diffusionsgeschwindigkeit durch die Temperatur beeinflußt.

Unter Knochenregenerationsmaterial werden im Rahmen der vorliegenden Erfindung alle zur Knochenregeneration geeigneten bioresorbierbaren Materialien aus der Gruppe der Erdalkali- und Alkali-Erdalkaliphosphate verstanden, insbesondere Calciumphosphate. Die konkrete Materialzusammensetzung des bioresorbierbaren Knochenregenerationsmaterials, z.B. Calciumphosphates ist für das erfindungsgemäße selbsthärtende, bioresorbierbare Kompositmaterial von untergeordneter Bedeutung im Vergleich zu dessen innerer Oberfläche und Porenstruktur.

Der Polymerisationsinitiator wird für seine Immobilisierung in gelöster Form mit einer ersten Teilmenge des bioresorbierbaren Knochenregenerationsmaterials wie Calciumphosphat in einem Mengenanteil von beispielsweise 0,1 bis 20 Masse-% vermischt und die Konzentration seiner Lösung so eingestellt, dass das Knochenregenerationsmaterial die Lösung des Polymerisationsinitiators vollständig in sein interkonnektierendes Porensystem aufsaugt. Anschließend wird das Knochenregenerationsmaterial getrocknet und steht so für weitere Herstellungsschritte, wie Konfektionierung und Sterilisation zur Verfügung. Nach der Trocknung des mit der Lösung des Polymerisationsinitiators getränkten Knochenregenerations-materials füllt dieser die Porensysteme des Knochen-regenerationsmaterials ganz oder wenigstens teilweise aus.

Als Polymerisationsinitiator haben sich Materialien aus der Gruppe der organischen Peroxide, vorzugsweise Dibenzoylperoxid, Lauroylperoxid und/oder Acetonperoxid besonders bewährt. Geeignete Lösungsmittel für den Polymerisationsinitiator sind verschiedene Ketone, vorzugsweise Aceton. Sie zeichnen sich einerseits durch gutes Lösungsverhalten, andererseits durch ein gutes Trocknungsverhalten aus und lassen sich restlos aus dem Porensystem des Knochenregenerationsmaterials, z.B. des Calciumphosphates entfernen, ohne das Reaktionsverhalten des Polymerisationsinitiators zu beeinträchtigen.

Der Polymerisationsaktivator wird in analoger Weise immobilisiert, indem dieser in einem organischen Lösungsmittel gelöst wird, oder indem er geschmolzen wird, wobei er beispielsweise in einer Menge von 0,1 bis 20 Masse-% bezogen auf eine zweite Teilmenge des Knochenregenerationsmaterials, z. B. Calciumphosphates, mit diesem vermischt wird. Die Konzentration der Lösung wird dabei so eingestellt, dass diese ebenfalls vollständig in das Porensystem des Knochenregenerationsmaterials eingesaugt wird. Anschließend wird das Knochenregenerations-material getrocknet und steht für weitere Herstellungsstufen, wie Konfektionierung und Sterilisation zur Verfügung. Nach der Trocknung des mit der Lösung des Polymerisationsaktivators getränkten Knochenregenerationsmaterials füllt dieser die Porensysteme des Knochenregenerationsmaterials ganz oder wenigstens teilweise aus.

Geeignete Lösungsmittel finden sich z.B. im Stand der Technik.

Als Polymerisationsaktivator haben sich Materialien aus der Gruppe N,N-Bis-(2-hydroxyethyl)-p-toluidin, N,N-Dimethyl-p-toluidin, N,N-Dimethyl-N,N-anilin, Ascorbinsäure und Barbitursäure einzeln oder als Mischung bewährt. Geeignete Lösungsmittel für den Polymerisationsaktivator sind verschiedene Alkohole oder Ketone, vorzugsweise Ethanol. Sie zeichnen sich einerseits durch gutes Lösungsverhalten, andererseits durch ein gutes Trocknungsverhalten aus und.lassen sich restlos aus dem Mikroporensystem des Calciumphosphates entfernen, ohne das Reaktionsverhalten des Polymerisationsaktivators zu beeinträchtigen.

Geeignete Knochenregenerationsmaterialien wie beispielsweise Calciumphosphate sind für die Knochenregeneration erfolgreich angewendete, bioresorbierbare Materialien aus der Gruppe der Erdalkaliphosphate oder der Alkali-Erdalkaliphosphate, insbesondere ihrer Orthophosphate, wie Alpha-Tricalciumphosphat oder Beta-Tricalciumphosphat, Magnesiumphosphat, Calcium-Natrium-Orthophosphat, calciumdefizitäre, carbonathaltige Hydroxylapatite, Octacalciumphosphat, Calciumhydrogenphosphat und/oder Calciumpyrophosphat. Die verwendete erste Teilmenge des Knochenregenerationsmaterials zur Immobilisierung des Polymerisationsinitiators kann chemisch und mineralogisch identisch der zweiten Teilmenge zur Immobilisierung des Polymerisationsaktivators sein. Die beiden Teilmengen können aber auch unterschiedlicher chemischer oder mineralogischer Natur sein, wenn dies die Eigenschaften des selbsthärtenden, bioresorbierbaren Kompositmaterials nicht negativ, vielmehr gar günstig beeinflusst.

Das interkonnektierende Porensystem der bioresorbierbaren Knochenregenerationsmaterialien, z.B. Calciumphosphate weist bevorzugt Porenquerschnitte im Bereich von 0,1 bis 100 µm, vorzugsweise im Bereich von 0,1 bis 10 *µ*m auf. Die Korngrößenverteilung der bioresorbierbaren Knochenregenerationsmaterialien wirkt sich dabei so auf die Freisetzung des Polymerisations-initiators und des Polymerisationsaktivators aus, dass mit zunehmender Korngröße die Freisetzungsgeschwindigkeit, und damit die Polymerisationsgeschwindigkeit verlangsamt wird. Erfindungsgemäß haben sich für die gewünschten Reaktionszeiten Korngrößen (als d₅₀-Werte) in einem Bereich von 1 bis 500 µm, vorzugsweise von 5 bis 300 µm bewährt. Das Knochenregenerationsmaterial, z.B. Calciumphosphat kann dabei in kristalliner, glasig-kristalliner oder amorpher Form eingesetzt werden. Die Materialmodifikation ist für die Immobilisierungsaufgabe von untergeordneter Bedeutung im Vergleich zur interkonnektierenden Porenstruktur und Korngrößenverteilung der Knochenregenerationsmaterial-Partikel.

Die interkonnektierende Porenstruktur des Knochenregenerations-materials, z.B. Calciumphosphates weist vorteilhaft eine hohe innere Oberfläche bei kleinen Poren-/Kanaldurchmessern auf. Eine bevorzugte Ausführungsform weist eine Oberfläche nach BET von wenigstens 0,1 m²/g bei mittleren Porendurchmessern im Bereich von 0,1 bis 20 *µ*m auf.

Zur Modifizierung ihrer Eigenschaften können den Knochenregenerationsmaterialien, z.B. Calciumphosphaten weitere biokompatible, eigenschafts- und/oder strukturmodifizierende Bestandteile zugesetzt werden, z.B. Siliziumverbindungen.

Der polymere Anteil des selbsthärtenden, bioresorbierbaren Kompositmaterials wird aus einem, zur Bildung eines biokompatiblen, bioresorbierbaren Polymernetzwerkes befähigten, flüssigen oder pastösen Monomer oder einer Mischung von derartigen Monomeren und deren Polymerisation unter Einsatz der o.g. Polymerisationsinitiatoren und Polymerisationsaktivatoren hergestellt. Erfindungsgemäß besteht dieses flüssige oder pastöse Monomer oder die Monomermischung aus einem Material der Gruppe der mehrfunktionellen, methacrylat-terminierten Oligomere, bevorzugt auf Basis von Milchsäure und/oder Glykolsäure und/oder δ-Hydroxyvaleriansäure und/oder ε-Hydroxycapronsäure und/oder Trimethylencarbonat.

Das selbsthärtende, bioresorbierbare Kompositmaterial, wird aus 40 bis 80 Masse-% eines Knochenregenerationsmaterials, z.B. Calciumphosphat oder Calciumphosphat-Mischung, und 20 bis 60 Masse-% eines flüssigen oder pastösen, bioresorbierbaren, mehrfunktionellen Monomers oder einer Monomermischung und gegebenenfalls, weiteren, die Eigenschaften des Monomers modifizierenden Bestandteilen gebildet. Seine Rezeptur besteht aus einer Mischung wenigstens einer Ausgangskomponente A, bestehend aus einem interkonnektierend-porösen Knochenregenerationsmaterial, z.B. Calciumphosphat, in dessen Porensystem ein Polymerisationsinitiator immobilisiert ist, einer Ausgangskomponente B, bestehend aus einem interkonnektierend-porösen Knochenregenerationsmaterial, z.B. Calciumphosphat, in dessen Porensystem ein Polymerisationsaktivator immobilisiert ist und einer Ausgangskomponente C, bestehend aus einem flüssigen oder pastösen, bioresorbierbaren, mehrfunktionellen Monomer oder einer entsprechenden Monomermischung und gegebenenfalls weiteren, die Eigenschaften des Monomers oder der Monomermischung modifizierenden Bestandteilen.

Solche eigenschaftsmodifizierenden Bestandteile sind beispielsweise Substanzen, die die Viskosität des Monomers, Monomergemischs und/oder deren Mischung mit dem Knochenregenerationsmaterial in einer für die Verarbeitung und für die Applikation wünschenswerten Weise verändern. Beispiele für geeignete viskositätsverändernde Substanzen sind oligomere oder polymere Derivate von alpha-Hydroxycarbonsäuren, insbesondere von Milch- und Glycolsäure sowie deren Copolymere und/oder Oligo- und Polyethylenglykole. Als viskositätserhöhende Substanz ist Dianhydro-D-glucit-bis-(poly-D,L-lactid) der Molmasse ca. 17.000 g/mol besonders geeignet. Weitere eigenschaftsmodifizierende Bestandteile sind wasserlösliche Substanzen oder Substanzen, die mit Wasser zu wasserlöslichen Folgeprodukten reagieren und die in einem wasserhaltigen Medium eine pH-Wert-Veränderung bewirken, wodurch die Abbaugeschwindigkeit des selbstgehärteten Kompositmaterials modifiziert werden kann. Eigenschaftsmodifizierende Bestandteile sind auch wasserlösliche Substanzen, die in partikulärer Form dem Monomer, Monomergemisch und/oder dessen Mischung mit dem Knochenregenerationsmaterial zugemischt werden und die nach Einbringung des selbstgehärteten Kompositmaterials in ein wasserhaltiges Medium, beispielsweise einen knöchernen Defekt, aus dem Kompositmaterial herausgelöst werden, wobei zusätzliche Poren entstehen. Auf diese Weise kann das Einwachsen von Zellen in das resorbierbare Kompositmaterial und dessen Degradation beschleunigt werden. Als besonders geeigneter Porenbildner kann partikuläres Natriumhydrogencarbonat eingesetzt werden. Eigenschaftsmodifizierende Bestandteile sind ferner Haftvermittler, die die Haftung zwischen dem selbstgehärtetem Kompositmaterial und dem natürlichem Gewebe, insbesondere Hartgewebe verbessern. Geeignete Haftvermittler sind insbesondere solche, die über freie Hydroxylgruppen verfügen wie z.B. Methacrylsäure-2-hydroxyethylester.

Eigenschaftsmodifizierende Bestandteile sind ebenfalls Farbstoffe und/oder Kontrastmittel, die beispielsweise dazu dienen, eine Visualisierung des Kompositmaterials im Körper zu erleichtern. Eigenschaftmodifizierende Bestandteile sind weiterhin pharmazeutische Wirkstoffe oder Wirkstoffgemische, die nach der Implantation aus dem Kompositmaterial freigestzt werden und zur lokalen therapeutischen oder prophylaktischen Behandlung des in der Umgebung des Kompositmaterials befindlichen Gewebes eingesetzt werden können. Beispiele für zumischbare Wirkstoffe sind Antibiotika, Antiinflammativa, proteinogene Wachstumsfaktoren oder Cancerostatika.

Das selbsthärtende, bioresorbierbare Kompositmaterial wird durch Polymerisation einer Mischung einer Ausgangskomponente A, bestehend aus einem interkonnektierend-porösen Knochenregenerationsmaterial, z.B. Calciumphosphat, in dessen Porensystem ein Polymerisationsinitiator immobilisiert ist, einer Ausgangskomponente B, bestehend aus einem interkonnektierend-porösen Knochenregenerationsmaterial z.B. Calciumphosphat, in dessen Porensystem ein Polymerisationsaktivator immobilisiert ist und einer Ausgangskomponente C, bestehend aus einem flüssigen oder pastösen, bioresorbierbaren, mehrfunktionellen Monomer oder einer mehrfunktionellen Monomermischung hergestellt. Optional können weitere, die Eigenschaften des Monomers, der Monomermischung oder des selbsthärtenden, bioresorbierbaren Kompositmaterials selbst modifizierende Bestandteile enthalten sein.

Das selbsthärtende, bioresorbierbare Kompositmaterial wird dabei aus 40 bis 80 Masse-% Knochenregenerationsmaterial, z.B. Calciumphosphat, und 20 bis 60 Masse-% eines flüssigen oder pastösen, bioresorbierbaren, mehrfunktionellen Monomers oder einer mehrfunktionellen Monomermischung und gegebenenfalls, weiteren, die Eigenschaften des Monomers modifizierenden Bestandteilen gebildet.

Die Verwendung des selbsthärtenden, bioresorbierbaren Kompositmaterials erfolgt je nach Vorgehensweise im Rahmen der Knochenheilung in zwei Richtungen. Bei Nutzung einer In-vivo-Polymerisation ist eine Anwendung als selbsthärtender, bioresorbierbarer Knochenkleber möglich, während bei einer Invitro-Polymerisation aus kompakten Kompositkörpern durch geeignete Bearbeitungsverfahren Implantate erzeugt werden können.

Der bioresorbierbare Knochenkleber dient vorzugsweise zur Fixierung von Splitterfrakturen im wenig oder nicht belasteten Skelettbereich und kann, kombiniert mit Osteosynthesemaßnahmen, im gesamten Skelettbereich Verwendung finden.

Mit Hilfe des erfindungsgemäßen Knochenklebers ausgehärtete Materialien, z.B. in Form zylindrischer Körper, eignen sich zur Fertigung von Implantaten für die Knochenregeneration. Sie sind sehr gut und mit hoher Genauigkeit maschinell zu bearbeiten. Es können sowohl Formkörper in standardisierten Abmessungen als auch patientenindividuelle Formkörper hergestellt werden, die als bioresorbierbare Implantate im Rahmen der Knochenheilung einsetzbar sind.

### Die Erfindung wird nachfolgend an Beispielen erläutert.

Für die Herstellung des selbsthärtenden, bioresorbierbaren Kompositmaterials kommen drei Ausgangskomponenten A, B und C sowie optional zur Verbesserung der Verarbeitungseigenschaften und/oder der physikalischen Eigenschaften des selbsthärtenden, bioresorbierbaren Kompositmaterials geeignete Haftvermittler und Verdicker zur Anwendung:
- Ausgangskomponente A:: Phasenreines β-Tricalciumphosphat, Korngröße < 50 µm, 0,2 m²/g Oberfläche nach BET, die Porosität liegt zu 90% im Durchmesserbereich 2 ± 1 *µ*m und enthält immobilisierten Polymerisationsinitiator
- Ausgangskomponente B:: Phasenreines β-Tricalciumphosphat, Korngröße < 50 µm, 0,2 m²/g Oberfläche nach BET, die Porosität liegt zu 90% im Durchmesserbereich 2 ± 1 µm und enthält immobilisierten Polymerisationsaktivator
- Ausgangskomponente C:: Bifunktionelles Monomer Dianhydro-D-glucit-bis-[(oligo-L-lactyl)-methacrylat]
(theoretisch 2 L-Milchsäure-Einheiten pro Hydroxylgruppe des Dianhydro-D-glucit)
- Haftvermittler:: Methacrylsäure-2-hydroxyethylester (HEMA)
- Verdicker:: Dianhydro-D-glucit-poly-D,L-lactid, Mₜₕ= 17442 g/mol

### 1. Herstellung der Ausgangskomponente A:

Es werden 1,706 g Dibenzoylperoxid (phlegmatisiert mit 25 Masse-% Wasser) in 15 ml Aceton gelöst. Diese Lösung wird mit 38,72 g CERASORB (ß-Tricalciumphosphat, 0,2 m²/g, Porendurchmesser 90% bei 2 ± 1 *µ*m) vermischt. Dabei saugt das CERASORB die Lösung vollständig in die Porensysteme auf. Nach Verdampfung des Acetons erhält man ein rieselfähiges Pulver.

### 2. Herstellung der Ausgangskomponente B:

Es werden 1,280 g N,N-Bis-(2-hydroxyethyl)-p-toluidin in 15 ml Ethanol gelöst. Diese Lösung wird mit 38,72 g CERASORB (β-Tricalciumphosphat, 0,2 m²/g, Porendurchmesser 90% bei 2 ± 1 µm) vermischt. Dabei saugt das CERASORB die Lösung vollständig in die Porensysteme auf. Nach Verdampfung des Ethanols erhält man ein rieselfähiges Pulver.

### 3. Herstellung der Ausgangskomponente C:

Ein Gemisch aus 18,26 g (0,125 mol) Dianhydro-D-glucitol, 36,02 g (0,25 mol) L-Lactid und 220 mg Zinn(II)-2-ethylhexanoat wird unter Feuchtigkeitsausschluß 4 Stunden bei 140°C gerührt. Nach Abkühlen des Gemisches auf Raumtemperatur erhält man einen transparenten zähen Feststoff, der in 120 ml Methylenchlorid gelöst und anschließend in 1200 ml Heptan umgefällt wird. Das gereinigte Produkt löst man in 40 ml Methylenchlorid. Dazu werden 50,6 g (0,5 mol) Triethylamin gegeben. Unter Eiskühlung, Feuchtigkeitsausschluß und Rühren werden 39,2 g (0,375 mol) Methacrylsäurechlorid vorsichtig zugetropft. Anschließend läßt man das Reaktionsgemisch unter Rühren auf Raumtemperatur erwärmen und bei Raumtemperatur über Nacht stehen. Danach wird das Reaktionsgemisch durch Extraktion gereinigt, über Natriumsulfat getrocknet und mit 40 mg p-Methoxyphenol versetzt. Im Vakuumrotationsverdampfer wird bei einer Badtemperatur von 30-35°C das Methylenchlorid abgezogen. Nachfolgend entfernt man noch verbliebene letzte Spuren von Methylenchlorid im Ölpumpenvakuum. Man erhält ein hellgelbes Öl, Ausbeute: m= 38,7 g (54,3 %)
IR (cm⁻¹) : 3110 (νCH sp²), 2994 (νCH sp³), 2945 (νCH sp³), 2879 (νCH sp³), 1757 (C=O), 1722 (C=O), 1639 (C=C).

### 4. Herstellung des selbsthärtenden, bioresorbierbaren Kompositmaterials:

Mit den Ausgangskomponenten A und B wurden unter Verwendung Ausgangskomponente C, die selbsthärtenden, bioresorbierbaren Kompositmaterialien R1 bis R4 hergestellt (s. Tab. 1). Diese weisen eine Verarbeitungszeit von 2-10 Minuten auf und härten dann innerhalb von 30 bis 60 Sekunden aus.

Mit den selbsthärtenden, bioresorbierbaren Kompositmaterialien wurden Klebeversuche unter Verwendung von entfetteten Rinderknochen durchgeführt. Es wurden dazu quadratische Knochenplättchen (7 x 7 x 3 mm) mittig auf rechteckige Knochenplättchen (20 x 10 x 3 mm) geklebt. Nach 18 Stunden Lagerung der verklebten Knochenplättchen in Raumluft erfolgte die Bestimmung der Zugscherfestigkeit mit einer Zugprüfmaschine der Firma Instron. Weiterhin wurden mit den selbsthärtenden, bioresorbierbaren Kompositmaterialien unter Verwendung von Silikongummiformen zylinderförmige Probekörper (Höhe 10 mm, Durchmesser 10 mm) hergestellt. Die Druckfestigkeit dieser Probekörper wurde ebenfalls mit einer Zugprüfmaschine der Firma Instron bestimmt (s. **Tab. 2).**

**Tab. 1: Zusammensetzung der selbsthärtenden, bioresorbierbaren Kompositmaterialien R1-4**

| **Komposition** | **Zusammensetzung** |
|---|---|
| R1 | 25,0 Masse-% Ausgangskomponente A |
| | 25,0 Masse-% Ausgangskomponente B |
| | 36,0 Masse-% Ausgangskomponente C |
| | 9,0 Masse-% Haftvermittler |
| | 5,0 Masse-% Verdicker |
| R2 | 27,5 Masse-% Ausgangskomponente A |
| | 27,5 Masse-% Ausgangskomponente B |
| | 32,4 Masse-% Ausgangskomponente C |
| | 8,1 Masse-% Haftvermittler |
| | 4,5 Masse-% Verdicker |
| R3 | 30,0 Masse-% Ausgangskomponente A |
| | 30,0 Masse-% Ausgangskomponente B |
| | 28,8 Masse-% Ausgangskomponente C |
| | 7,2 Masse-% Haftvermittler |
| | 4,0 Masse-% Verdicker |
| R4 | 30,0 Masse-% Ausgangskomponente A |
| | 32,5 Masse-% Ausgangskomponente B |
| | 25,2 Masse-% Ausgangskomponente C |
| | 6,3 Masse-% Haftvermittler |
| | 3,5 Masse-% Verdicker |

**Tab.2: Zugscherfestigkeit der mit den selbsthärtenden, bioresorbierbaren Kompositmaterialien R1-4 verklebten Knochenplättchen und Druckfestigkeit der aus R1-4 hergestellten Probekörper.**

| **Komposition** | **Zugscherfestigkeit [MPa]** | **Druckfestigkeit [MPa]** |
|---|---|---|
| R1 | 13,9 ± 8,1 | 74,4 ± 6,1 |
| R2 | 5,5 ± 2,6 | 78,1 ± 5,0 |
| R3 | 11,0 ± 2,8 | 83,9 ± 5,4 |
| R4 | 16,6 ± 8,3 | 79,1 ± 13,8 |

## Patentansprüche

1. Verfahren zur Herstellung eines selbsthärtenden bioresorbierbaren Kompositmaterials, bei dem man
(i) einen Polymerisationsinitiator mit Hilfe einer ersten Teilmenge eines interkonnektierend-porösen bioresorbierbaren anorganischen Knochenregenerationsmaterials immobilisiert,
(ii) einen Polymerisationsaktivator mit Hilfe einer zweiten Teilmenge des Knochenregenerationsmaterials gemäß (i) oder eines anderen interkonnektierend-porösen bioresorbierbaren anorganischen Knochenregenerationsmaterials immobilisiert,
(iii) die bei den Stufen (i) und (ii) anfallenden Komponenten mit einem flüssigen oder pastösen zu einem biokompatiblen, und bioresorbierbaren Polymer polymerisierbaren mehrfunktionellen Monomer oder mit einer flüssigen oder pastösen Mischung zu einem biokompatiblen und bioresorbierbaren Polymer polymerisierbarer mehrfunktioneller Monomerer mischt und
(iv) das mit der erhaltenen Mischung vermischte Monomer oder Monomergemisch polymerisiert und das Kompositmaterial gewinnt.

2. Verfahren nach Anspruch 1, bei dem man Bestandteile zumischt, insbesondere bei Stufe (iii), die die Eigenschaften des Monomer, des Monomergemischs und/oder des Kompositmaterials modifizieren.

3. Verfahren nach Anspruch 2, bei dem man einen oder mehrere modifizierende Bestandteile zumischt, die aus der Gruppe ausgewählt sind von: Verdickungsmittel, Verdünnungsmittel, polymerer Füllstoff, Porogen, pH-modifizierende Substanz, Farbstoff und Adhäsionsvermittler.

4. Verfahren nach mindestens einem der vorhergehenden Ansprüche, **dadurch gekennzeichnet, dass** es sich bei mindestens einer der zugemischten Bestandteile um eine Substanz handelt, die die Viskosität des Monomers, Monomergemischs und/oder deren Mischung mit dem Knochenregenerationsmaterial verändert.

5. verfahren nach Anspruch 4, **dadurch gekennzeichnet, dass** es sich bei den die Viskosität des Monomers, Monomergemischs und/oder deren Mischung mit dem Knochenregenerationsmaterial verändernden Substanzen um oligomere oder polymere Derivate von alpha-Hydroxycarbonsäuren, vorzugsweise solchen der Milch- und/oder Glycolsäure, und/oder um Substanzen aus der Gruppe der Oligo- oder Polyethylenglykole handelt.

6. Verfahren nach Anspruch 4 und/oder 5, **dadurch gekennzeichnet, dass** Dianhydro-D-glucit-bis-(poly-D,L-lactid) als viskositätsverändernde Substanz eingesetzt wird.

7. Verfahren nach mindestens einem der vorhergehenden Ansprüche, **dadurch gekennzeichnet, dass** es sich bei mindestens einer der zugemischten Bestandteile um eine wasserlösliche oder mit Wasser zu wasserlöslichen Folgeprodukten reagierenden Substanz handelt, die in einem wasserhaltigen Medium eine pH-Wert-Änderung bewirkt.

8. Verfahren nach mindestens einem der vorhergehenden Ansprüche, **dadurch gekennzeichnet, dass** es sich bei mindestens einer der zugemischten Bestandteile um eine wasserlösliche porenbildende Substanz handelt, die dem Monomer, Monomergemisch und/oder deren Mischung mit dem Knochenregenerationsmaterial in partikulärer Form zugesetzt wird.

9. Verfahren nach Anspruch 7 und/oder 8, **dadurch gekennzeichnet, dass** Natriumhydrogencarbonat als wasserlösliche pH-Wert-ändernde und porenbildende Substanz verwendet wird.

10. Verfahren nach mindestens einem der vorhandenen Ansprüche, **dadurch gekennzeichnet, dass** es sich bei mindestens einem der zugemischten Bestandteile um eine Substanz handelt, die als Haftvermittler zwischen Kompositmaterial und lebendem Gewebe, vorzugsweise Hartgewebe, wirkt.

11. Verfahren nach Anspruch 10, **dadurch gekennzeichnet, dass** hydroxylgruppenhaltige Haftvermittler, vorzugsweise Methacrylsäure-2-hydroxyethylester als Haftvermittler verwendet werden.

12. Verfahren nach mindestens einem der vorhandenen Ansprüchen, **dadurch gekennzeichnet, dass** es sich bei mindestens einer der zugemischten Bestandteile um einen Farbstoff oder ein Kontrastmittel handelt.

13. Verfahren nach mindestens einem der vorhergehenden Ansprüche, **dadurch gekennzeichnet, dass** es sich bei mindestens einem der zugemischten Bestanteile um einen pharmazeutischen Wirkstoff oder ein Wirkstoffgemisch zur lokalen Therapie und/oder Prophylaxe handelt.

14. Verfahren nach Anspruch 13, **dadurch gekennzeichnet, dass** Antibiotika, Antiinflammativa, proteinogene Wachstumsfaktoren und/oder Cancerostatika als pharmazeutische Wirkstoffe verwendet werden.

15. Verfahren nach mindestens einem der vorhergehenden Ansprüche, bei dem man die erste Teilmenge und die zweite Teilmenge des Knochenregenerations-materials in einem Verhältnis von 1:10 bis 10:1 einsetzt und/oder den Polymerisationsinitiator und den Polymerisationsaktivator mit den jeweiligen Teilmengen des Knochenregenerationsmaterials in einem Verhältnis von 1:10 bis 10:1 immobilisiert (jeweils auf Massebasis).

16. Verfahren nach mindestens einem der vorhergehenden Ansprüche, bei dem man das Knochenregenerationsmaterial in Form von Pulver oder Granulat einsetzt.

17. Verfahren nach mindestens einem der vorhergehenden Ansprüche, bei dem man bei Stufe (i) gemäß Anspruch 1 das Knochenregenerationsmaterial mit einer Lösung des Polymerisationsinitiators versetzt, die Lösung das Knochenregenerationsmaterial infiltrieren lässt und danach das Knochenregenerationsmaterial trocknet.

18. Verfahren nach mindestens einem der vorhergehenden Ansprüche, bei dem man eine Lösung des Polymerisationsinitiators in einer Menge von 0,1 bis 20 Masse-% mit dem Knochenregenerationsmaterial vermischt (fester Initiator bezogen auf Knochencegenerationsmaterial).

19. Verfahren nach mindestens einem der vorhergehenden Ansprüche, bei dem man ein organisches Peroxid als Polymerisationsinitiator verwendet, vorzugsweise ein aus der Dibenzoylperoxid, Lauroylperoxid und Acetonperoxid umfassenden Gruppe ausgewähltes organisches Peroxid.

20. Verfahren nach mindestens einem der vorhergehenden Ansprüche, bei dem man bei Stufe (ii) gemäß Anspruch 1 das Knochenregenerationsmaterial mit einer Schmelze oder einer Lösung des Polymerisationsaktivators versetzt, die Lösung das Knochenregenerationsmaterials infiltrieren lässt und danach das Knochenregenerationsmaterial trocknet.

21. Verfahren nach mindestens einem der vorhergehenden Ansprüche, bei dem man eine Lösung des Polymerisationsaktivators in einer Menge von 0,1 bis 20 Masse-% mit dem Knochenregenerations-material vermischt (fester Aktivator bezogen auf Knochen-regenerationsmaterial).

22. Verfahren nach mindestens einem der vorhergehende Ansprüche, bei dem man einen oder mehrere Polymerisationsaktivatoren verwendet, die aus der N,N-Bis-(2-hydroxyethyl)-p-toluidin, N,N-Dimethyl-p-toluidin, N,N-Dimethyl-N,N-anilin, Ascorbinsäure und Barbitursäure umfassenden Gruppe ausgewählt sind.

23. Verfahren nach mindestens einem der vorhergehenden Ansprüche, bei dem man den Polymerisationsinitiator in Form einer Lösung und/oder den Polymerisationsaktivator in Form einer Lösung einsetzt und die Lösung(en) vom Knochenregenerationsmaterial vollständig aufsaugen läßt oder soweit wie möglich aufsaugen läßt und den nicht aufgesaugten Überschuß vor Stufe (iii) gemäß Anspruch 1 entfernt.

24. Verfahren nach mindestens einem der vorhergehenden Ansprüche, bei dem man ein Erdalkaliphosphat und/oder ein Alkali/Erdalkaliphosphat als anorganisches Knochenregenerationsmaterial verwendet, insbesondere ein Erdalkali-orthophosphat und/oder Alkali/Erdalkali-orthophosphat, vorzugsweise ein Knochenregenerationsmaterial, das aus der alpha-Tricalciumphosphat, beta-Tricalciumphosphat, calciumdefizitäreo carbonathaltigen Hydroxylapatit, Octacalciumphosphat, Magnesiumphosphat, Calciumhydrogenphosphat, Calcium/Natrium-orthophosphat und Calciumpyrophosphat umfassenden Gruppe ausgewählt ist.

25. Verfahren nach mindestens einem der vorhergehenden Ansprüche, bei man für die Immobilisierung des polymerisationsinitiators dasselbe Knochenregenerationsmaterial verwendet als für die Immobilisierung des Polymerisationsaktivators.

26. Verfahren nach mindestens einem der Ansprüche 1 bis 24, bei dem sich das Knochenregenerationsmateral für die Immobilisierung des Initiators und das Knochenregenerationsmateral für die Immobilisierung des Aktivators durch ihre chemische und/oder mineralogische Natur voneinander unterscheiden.

27. Verfahren nach mindestens einem der vorhergehenden Ansprüche, bei dem man ein interkonnektierend-poröses Knochenregenerationsmaterial, insbesondere Calciumphosphat, mit folgenden Kenndaten verwendet:
- Porendurchmesser von 0,1 bis 500 µm, vorzugsweise von 0,1 bis 100 µm, und/oder
- Korngrößen (d₅₀-Werte) von 1 bis 500 *µ*m, vorzugsweise 5 bis 300 µm, und/oder
- Oberfläche nach BET von wenigstens 0,1 m²_{/}g.

28. Verfahren nach mindestens einem der Ansprüche 1 bis 26, bei dem man ein interkonnektierend-poröses Knochenregenerationsmaterial, insbesondere Calciumphosphat, mit einem für den Polymerisationsinitiator und/oder den Polymerisationsaktivator zugänglichen Porenvolumen von 0,4 cm³/g oder mehr bei Erhalt der Integrität der Teilchen des Knochenregenerationsmaterials und insbesondere von 0,4 bis 3,3 cm³/g einsetzt.

29. Verfahren nach mindestens einem der vorhergehenden Ansprüche, bei dem man das Knochenregenerationsmaterial, insbesondere Calciumphosphat, in kristalliner, teilkristalliner, glasiger oder amorpher Form verwendet.

30. Verfahren nach mindestens einem der vorhergehenden Ansprüche, insbesondere nach Anspruch 2 und/oder 3, bei dem man dem Knochenregenerationsmaterial Bestandteile zumischt, die biokompatibel sind und die Eigenschaften des Regenerationsmaterials modifizieren, insbesondere Siliziumverbindungen.

31. Verfahren nach mindestens einem der vorhergehenden Ansprüche, bei dem man als Monomer oder als Monomere des Monomergemischs ein mehrfunktionelles Oligomeres mit endständigen Methacrylatgruppen verwendet, insbesondere ein Oligomeres von Milchsäure und/oder Glykolsäure und/oder delta-Hydroxyvaleriansäure und/oder epsilon-Hydroxycapronsäure und/oder Trimethylencarbonat.

32. selbsthärtende bioresorbierbares Kompositmaterial als Set, bestehend aus oder umfassend
(i) eine erste Teilmenge eines interkonnektierend-porösen bioresorbierbaren anorganischen Knochenregenerationsmaterials und einen Polymerisationsinitiator, der mit Hilfe dieser ersten Teilmenge immobilisiert ist,
(ii) eine zweite Teilmenge des Knochenregenerationsmaterials gemäß (i) oder eines anderen interkonnektierend-porösen bioresorbierbaren anorganischen Knochenregenerations-materials und einen Polymerisationeaktivator, der mit Hilfe dieser zweiten Teilmenge immobilisiert ist, und
(iii) ein flüssiges oder pastöses zu einem biokompatiblen und bioresorbierbaren Polymer polymerisierbares mehrfurktionelles Monomer oder eine flüssige oder pastöse Mischung zu einem biokompatiblen und bioresorbierbaren Polymer polymerisierbarer mehrfunktioneller Monomerer.

33. Kompositmaterial nach Anspruch 32 mit einem Masseverhältnis von Knochenregenerationsmaterial : Monomer oder Monomergemisch von 4 : 6 bis 7:3.

34. Kompositmaterial als Set nach Anspruch 32 und/oder 33, wobei die Komponenten (i), (ii) und (iii) gemäß einem Verfahren gemäß mindestens einem der Ansprüche 2 bis 31 erhältlich sind.

35. Verwendung eines selbsthärtenden Knochenregenerations-materials in Form eines Sets gemäß mindestens einem der Ansprüche 32 bis 34 zur Herstellung eines Knochenklebers für die Fixierung von Knochenfrakturen.

## Claims

1. A method of producing a self-hardening bioabsorbable composite material, wherein
(i) a polymerisation initiator is immobilised with the aid of a first partial amount of an interconnectingly porous bioabsorbable inorganic bone regeneration material,
(ii) a polymerisation activator is immobilised with the aid of a second partial amount of the bone regeneration material according to (i) or of a different interconnectingly porous bioabsorbable inorganic bone regeneration material,
(iii) the components obtained in steps (i) and (ii) are mixed with a liquid or paste-form multi-functional monomer capable of polymerisation to form a biocompatible and bioabsorbable polymer or with a liquid or paste-form mixture of multi-functional monomers capable of polymerisation to form a biocompatible and bioabsorbable polymer, and
(iv) the monomer or monomer mixture admixed with the mixture produced is polymerised and the composite material is obtained.

2. The method according to claim 1, wherein, especially in step (iii), constituents which modify the properties of the monomer, monomer mixture and/or composite material are mixed in.

3. The method according to claim 2, wherein one or more modifying constituents are mixed in which are selected from the group: thickeners, diluents, polymeric fillers, porogens, pH-modifying substances, colourants and adhesion-imparting agents.

4. The method according to at least one of the preceding claims, **characterised in that** at least one of the constituents mixed in is a substance which alters the viscosity of the monomer, the monomer mixture and/or the mixture thereof with the bone regeneration material.

5. The method according to claim 4, **characterised in that** the substances altering the viscosity of the monomer, the monomer mixture and/or the mixture thereof with the bone regeneration material are oligomeric or polymeric derivatives of alpha-hydroxycarboxylic acids, preferably those of lactic and/or glycolic acid, and/or are substances from the group of oligo- or poly-ethylene glycols.

6. The method according to claim 4 and/or 5, **characterised in that** dianhydro-D-glucitol-bis(poly-D,L-lactide) is used as viscosity-increasing substance.

7. The method according to at least one of the preceding claims, **characterised in that** at least one of the constituents mixed in is a substance which is water-soluble or which reacts with water to form water-soluble resultant products and which brings about a pH change in a water-containing medium.

8. The method according to at least one of the preceding claims, **characterised in that** at least one of the constituents mixed in is a water-soluble pore-forming substance which is added to the monomer, monomer mixture and/or the mixture thereof with the bone regeneration material in particulate form.

9. The method according to claim 7 and/or 8, **characterised in that** sodium hydrogen carbonate is used as water-soluble pH-modifying and pore-forming substance.

10. The method according to at least one of the present claims, **characterised in that** at least one of the constituents mixed in is a substance which acts as an adhesion-imparting agent between the composite material and living tissue, preferably hard tissue.

11. The method according to claim 10, **characterised in that** hydroxyl-group-containing adhesion-imparting agents, preferably methacrylic acid 2-hydroxyethyl ester, are used as adhesion-imparting agent.

12. The method according to at least one of the present claims, **characterised in that** at least one of the constituents mixed in is a colourant or a contrast agent.

13. The method according to at least one of the preceding claims, **characterised in that** at least one of the constituents mixed in is a pharmaceutical active ingredient or an active ingredient mixture for local therapy and/or prophylaxis.

14. The method according to claim 13, **characterised in that** antibiotics, anti-inflammatories, proteinogenic growth factors and/or cancerostatics are used as pharmaceutical active ingredients.

15. The method according to at least one of the preceding claims, wherein the first partial amount and the second partial amount of the bone regeneration material are used in a ratio of from 1:10 to 10:1 and/or the polymerisation initiator and the polymerisation activator are immobilised with the respective partial amounts of the bone regeneration material in a ratio of from 1:10 to 10:1 (based on weight in each case).

16. The method according to at least one of the preceding claims, wherein the bone regeneration material is used in the form of powder or granules.

17. The method according to at least one of the preceding claims, wherein, in step (i) according to claim 1, a solution of the polymerisation initiator is added to the bone regeneration material, the solution is allowed to infiltrate the bone regeneration material, and afterwards the bone regeneration material is dried.

18. The method according to at least one of the preceding claims, wherein a solution of the polymerisation initiator is mixed with the bone regeneration material in an amount of from 0.1 to 20 % by weight (solid initiator based on bone regeneration material).

19. The method according to at least one of the preceding claims, wherein an organic peroxide is used as polymerisation initiator, preferably an organic peroxide selected from the group comprising dibenzoyl peroxide, lauroyl peroxide and acetone peroxide.

20. The method according to at least one of the preceding claims, wherein, in step (ii) according to claim 1, a melt or solution of the polymerisation activator is added to the bone regeneration material, the solution is allowed to infiltrate the bone regeneration material, and afterwards the bone regeneration material is dried.

21. The method according to at least one of the preceding claims, wherein a solution of the polymerisation activator is mixed with the bone regeneration material in an amount of from 0.1 to 20 % by weight (solid activator based on bone regeneration material).

22. The method according to at least one of the preceding claims, wherein one or more polymerisation activators are used which are selected from the group comprising N,N-bis(2-hydroxyethyl)-p-toluidine, N,N-dimethyl-p-toluidine, N,N-dimethyl-N,N-aniline, ascorbic acid and barbituric acid.

23. The method according to at least one of the preceding claims, wherein the polymerisation initiator is used in the form of a solution and/or the polymerisation activator is used in the form of a solution and the solution(s) is/are allowed to be drawn up by the bone regeneration material completely or as far as possible and the excess not drawn up is removed before step (iii) according to claim 1.

24. The method according to at least one of the preceding claims, wherein there is used, as inorganic bone regeneration material, an alkaline earth metal phosphate and/or an alkali metal/alkaline earth metal phosphate, especially an alkaline earth metal orthophosphate and/or alkali metal/alkaline earth metal orthophosphate, preferably a bone regeneration material which is selected from the group comprising alpha-tricalcium phosphate, beta-tricalcium phosphate, calcium-deficient carbonate-containing hydroxyapatite, octacalcium phosphate, magnesium phosphate, calcium hydrogen phosphate, calcium/sodium orthophosphate and calcium pyrophosphate.

25. The method according to at least one of the preceding claims, wherein the same bone regeneration material is used for the immobilisation of the polymerisation initiator as for the immobilisation of the polymerisation activator.

26. The method according to at least one of claims 1 to 24, wherein the bone regeneration material for the immobilisation of the initiator and the bone regeneration material for the immobilisation of the activator differ from one another in their chemical and/or mineralogical nature.

27. The method according to at least one of the preceding claims, wherein an interconnectingly porous bone regeneration material, especially calcium phosphate, having the following characteristic data is used:
- pore diameters from 0.1 to 500 µm, preferably from 0.1 to 100 µm, and/or
- particle sizes (d₅₀ values) of from 1 to 500 µm, preferably from 5 to 300 µm, and/or
- BET surface area of at least 0.1 m²/g.

28. The method according to at least one of claims 1 to 26, wherein there is used an interconnectingly porous bone regeneration material, especially calcium phosphate, having a pore volume, accessible to the polymerisation initiator and/or the polymerisation activator, of 0.4 cm³/g or more, and especially from 0.4 to 3.3 cm³/g, while retaining the integrity of the particles of the bone regeneration material.

29. The method according to at least one of the preceding claims, wherein the bone regeneration material, especially calcium phosphate, is used in crystalline, partly crystalline, glassy or amorphous form.

30. The method according to at least one of the preceding claims, especially according to claim 2 and/or 3, wherein constituents which are biocompatible and which modify the properties of the regeneration material, especially silicon compounds, are mixed in with the bone regeneration material.

31. The method according to at least one of the preceding claims, wherein there is used, as the monomer or as monomers of the monomer mixture, a multi-functional oligomer having terminal methacrylate groups, especially an oligomer of lactic acid and/or glycolic acid and/or delta-hydroxyvaleric acid and/or epsilon-hydroxycaproic acid and/or trimethylene carbonate.

32. A self-hardening bioabsorbable composite material in the form of a set, consisting of or comprising:
(i) a first partial amount of an interconnectingly porous bioabsorbable inorganic bone regeneration material and a polymerisation initiator which is immobilised with the aid of that first partial amount,
(ii) a second partial amount of the bone regeneration material according to (i) or of a different interconnectingly porous bioabsorbable inorganic bone regeneration material and a polymerisation activator which is immobilised with the aid of that second partial amount, and
(iii) a liquid or paste-form multi-functional monomer capable of polymerisation to form a biocompatible and bioabsorbable polymer or a liquid or paste-form mixture of multi-functional monomers capable of polymerisation to form a biocompatible and bioabsorbable polymer.

33. The composite material according to claim 32, having a weight ratio of bone regeneration material : monomer or monomer mixture of from 4 : 6 to 7 : 3.

34. The composite material in the form of a set according to claim 32 and/or 33, wherein the components (i), (ii) and (iii) are obtainable by a method according to at least one of claims 2 to 31.

35. Use of a self-hardening bone regeneration material in the form of a set according to at least one of claims 32 to 34 in producing a bone adhesive for the fixing of bone fractures.

## Revendications

1. Procédé pour la fabrication d'un matériau composite biorésorbable, dans lequel
(i) on immobilise un initiateur de polymérisation à l'aide d'une première quantité partielle d'un matériau de régénération des os s'interconnectant, poreux, biorésorbable, inorganique,
(ii) on immobilise un activateur de polymérisation à l'aide d'une deuxième quantité partielle d'un matériau de régénération des os s'interconnectant, poreux, biorésorbable, inorganique,
(iii) on mélange les composants résultants des étapes (i) et (ii) avec un monomère multifonctionnel liquide ou pâteux pouvant être polymérisé en un polymère biocompatible et biorésorbable ou avec un mélange liquide ou pâteux d'un monomère multifonctionnel pouvant être polymérisé en un polymère biocompatible et biorésorbable, et
(iv) on polymérise le monomère, ou le mélange de monomères, ajouté au mélange obtenu et on obtient le matériau composite.

2. Procédé selon la revendication 1 dans lequel on ajoute des composants au mélange, en particulier dans l'étape (iii), qui modifient les propriétés du monomère, du mélange de monomères et/ou du matériau composite.

3. Procédé selon la revendication 2 dans lequel on mélange un ou plusieurs composants modificateurs, qui sont choisis dans le groupe des : épaississants, diluants, charges polymériques, substances générant des pores, substances modifiant le pH, colorants et promoteurs d'adhésion.

4. Procédé selon au moins l'une des revendications précédentes **caractérisé en ce que** pour au moins l'un des composants mélangés, il s'agit d'une substance qui modifie la viscosité du monomère, du mélange de monomères et/ou de leur mélange avec le matériau de régénération de l'os.

5. Procédé selon la revendication 4 **caractérisé en ce que**, pour les substances modifiant la viscosité du monomère, du mélange de monomères et/ou de leur mélange avec le matériau de régénération des os, il s'agit d'oligomères ou de dérivés de polymères d'acides alpha-hydroxy carboxyliques, de préférence ceux des acides lactique et/ou glycolique, et/ou de substances faisant partie du groupe des oligo- ou des poly-éthylèneglycols.

6. Procédé selon la revendication 4 et/ou 5 **caractérisé en ce que** du dianhydro-D-glucide-bis-(poly-D,L-lactose) est employé en tant que substance modifiant la viscosité.

7. Procédé selon au moins l'une des revendications précédentes **caractérisé en ce qu'**il s'agit, pour au moins l'un des composants mélangés, d'une substance réagissant en donnant des produits dérivés solubles dans l'eau, ou pouvant être rendus solubles avec de l'eau, qui provoque une modification de la valeur du pH dans un milieu contenant de l'eau.

8. Procédé selon au moins l'une des revendications précédentes **caractérisé en ce qu'**il s'agit d'une substance formant des pores, soluble dans l'eau, pour au moins l'un des composants mélangés, qui est ajouté au monomère, au mélange de monomères et/ou à leur mélange avec le matériau de régénération des os sous forme particulaire.

9. Procédé selon la revendication 7 et/ou 8, **caractérisé en ce que** de l'hydrogénocarbonate de sodium est utilisé en tant que substance modifiant la valeur du pH et formant des pores.

10. Procédé selon au moins l'une des revendications présentes **caractérisé en ce qu'**il s'agit d'une substance, qui agit en tant que promotrice d'adhésion entre un matériau composite et du tissu vivant, de préférence du tissu dur, pour au moins l'un des composants mélangés.

11. Procédé selon la revendication 10 **caractérisé en ce que** des promoteurs d'adhésion contenant des groupes hydroxyles, de préférence du 2-hydroxyéthylester de l'acide méthacrylique, sont employés en tant que promoteurs d'adhésion.

12. Procédé selon au moins l'une des revendications présentes **caractérisé en ce qu'**il s'agit d'un colorant ou d'un agent de contraste pour au moins l'un des composants mélangés.

13. Procédé selon au moins l'une des revendications précédentes **caractérisé en ce qu'**il s'agit d'une matière active pharmaceutique ou d'un mélange de matières actives pour une thérapie locale et/ou une prophylaxie, pour au moins l'un des composants mélangés.

14. Procédé selon la revendication 13 **caractérisé en ce que** des antibiotiques, des anti-inflammatoires, des facteurs de croissance protéinogènes et/ou des cancérostatiques sont utilisés en tant que matières actives pharmaceutiques.

15. Procédé selon au moins l'une des revendications précédentes dans lequel on utilise la première quantité partielle et la deuxième quantité partielle du matériau de régénération des os dans un rapport de 1 : 10 à 10 : 1 et/ou on immobilise l'initiateur de polymérisation et l'activateur de polymérisation avec des quantités respectives de matériau de régénération des os dans un rapport de 1 : 10 à 10 : 1 (chaque fois sur la base de la masse).

16. Procédé selon au moins l'une des revendications précédentes dans lequel le matériau de régénération des os est employé sous la forme de poudre ou de granulés.

17. Procédé selon au moins l'une des revendications précédentes dans lequel, dans l'étape (i), selon la revendication 1, on ajoute le matériau de régénération des os à une solution de l'initiateur de polymérisation, on laisse la solution s'infiltrer dans le matériau de régénération des os et ensuite on sèche le matériau de régénération des os.

18. Procédé selon au moins l'une des revendications précédentes dans lequel on mélange une solution de l'initiateur de polymérisation avec le matériau de régénération des os en une quantité de 0,1 à 20 % en masse (initiateur sec par rapport au produit de régénération des os).

19. Procédé selon au moins l'une des revendications précédentes dans lequel on emploie un peroxyde organique en tant qu'initiateur de polymérisation, de préférence un produit faisant partie du groupe comprenant un peroxyde organique choisi parmi le peroxyde de dibenzyle, le peroxyde de lauroyle et le peroxyde d'acétone.

20. Procédé selon au moins l'une des revendications précédentes dans lequel, dans l'étape (ii) selon la revendication 1, on ajoute le matériau de régénération des os à l'activateur de polymérisation fondu ou en solution, on laisse la solution s'infiltrer dans le matériau de régénération des os et ensuite on sèche le matériau de régénération des os.

21. Procédé selon au moins l'une des revendications précédentes dans lequel on mélange une solution de l'activateur de polymérisation en une quantité de 0,1 à 20 % en masse avec le matériau de régénération des os (activateur sec par rapport au matériau de régénération des os).

22. Procédé selon au moins l'une des revendications précédentes dans lequel on emploie un ou plusieurs activateurs de polymérisation qui sont choisis dans le groupe comprenant la N,N-bis-(2-hydroxyéthyl)-p-toluidine, la N,N-diméthyl-p-toluidine, la N,N-diméthyl-N,N-aniline, l'acide ascorbique et l'acide barbiturique.

23. Procédé selon au moins l'une des revendications précédentes dans lequel on emploie l'initiateur de polymérisation sous la forme d'une solution et/ou l'activateur de polymérisation sous la forme d'une solution et on laisse la(les) solution(s) du matériau de régénération des os tout absorber ou absorber tout ce qu'il est possible d'absorber, et on enlève le surplus non absorbé avant de l'étape (iii) selon le revendication 1.

24. Procédé selon au moins l'une des revendications précédentes dans lequel on emploie un phosphate d'alcalino-terreux et/ou un phosphate alcalin/alcalino-terreux en tant que matériau de régénération des os, en particulier un ortho phosphate alcalino-terreux et/ou un ortho phosphate alcalin/alcalino-terreux, de préférence un matériau de régénération des os qui est choisi dans le groupe comportant l'alpha-phosphate tricalcique, le bêta-phosphate tricalcique, l'hydroxy apatite contenant du carbone déficitaire en calcium, l'octacalciumphosphate, le phosphate de magnésium, l'hydrogéno-phosphate de calcium, l'othophosphate de calcium/sodium et le pyrophosphate de calcium.

25. Procédé selon au moins l'une des revendications précédentes dans lequel on emploie pour l'immobilisation de l'initiateur de polymérisation le même matériau de régénération des os que pour l'immobilisation de l'activateur de polymérisation.

26. Procédé selon au moins l'une des revendications de 1 à 24 dans lequel le matériau de régénération des os pour l'immobilisation de l'initiateur et le matériau de régénération des os pour l'immobilisation de l'activateur se différencient l'un de l'autre par leur nature chimique et/ou minéralogique.

27. Procédé selon au moins l'une des revendications précédentes dans lequel on emploie un matériau de régénération des os s'interconnectant, poreux, en particulier du phosphate de calcium, avec les propriétés suivantes :
- diamètre des pores de 0,1 à 500 µm, de préférence de 0,1 à 100 µm, et/ou
- taille des grains (valeurs d₅₀) de 1 à 500 µm, de préférence de 5 à 300 µm, et/ou
- surface selon la méthode BET d'au moins 0,1 m²/g.

28. Procédé selon au moins l'une des revendications de 1 à 26 dans lequel on emploie un matériau de régénération des os s'interconnectant, poreux, en particulier du phosphate de calcium, comportant un volume poreux disponible pour l'initiateur de polymérisation et/ou pour l'activateur de polymérisation de 0,4 cm³/g ou plus, et, en particulier, de 0,4 à 3,3 cm³/g, tout en conservant l'intégrité des particules du matériau de régénération des os.

29. Procédé selon au moins l'une des revendications précédentes dans lequel on emploie le matériau de régénération des os, en particulier, le phosphate de calcium, sous forme cristalline, sous forme partiellement cristalline, sous forme de verre ou sous forme amorphe.

30. Procédé selon au moins l'une des revendications précédentes, en particulier selon la revendication 2 et/ou 3, dans lequel on mélange des composants qui sont biocompatibles avec le matériau de régénération des os et qui modifient les propriétés du matériau de régénération des os, en particulier, des composés de silicium.

31. Procédé selon au moins l'une des revendications précédentes dans lequel on emploie, en tant que monomère ou en tant que monomères du mélange de monomères, un oligomère multifonctionnel avec des groupes méthacrylates en dernière position, en particulier, un oligomère de l'acide lactique, et/ou de l'acide glycolique, et/ou de l'acide delta-hydroxy valérique, et/ou de l'acide epsilon-hydroxy caproïque, et/ou du triméthylène carbonate.

32. Matériau composite biorésorbable auto-durcissant sous forme d'un ensemble constitué par, ou comprenant
(i) une première quantité partielle d'un matériau de régénération des os s'interconnectant, poreux, biorésorbable, inorganique et un initiateur de polymérisation qui est immobilisé à l'aide de cette première quantité partielle,
(ii) une seconde quantité partielle de matériau de régénération des os selon (i) ou un autre matériau de régénération des os s'interconnectant, poreux, biorésorbable, inorganique et un activateur de polymérisation qui est immobilisé à l'aide ce cette seconde quantité partielle, et
(iii) un monomère multifonctionnel liquide ou pâteux pouvant être polymérisé en un polymère biocompatible et biorésorbable ou un mélange des monomères multifonctionnelles pouvant être liquide ou pâteux polymérisé en un polymère, biocompatible et résorbable.

33. Matériau composite selon la revendication 32 comportant un rapport en matériau de régénération des os : monomère ou en mélange de monomères de 4 : 6 à 7 : 3.

34. Matériau composite d'après l'ensemble selon la revendication 32 et/ou 33, les composants (i), (ii) et (iii) étant obtenus d'après un procédé selon au moins l'une des revendications de 2 à 31.

35. Utilisation d'un matériau de régénération des os auto-durcissant sous la forme d'un ensemble selon au moins l'une des revendications de 32 à 34 pour la fabrication d'une colle pour les os pour la fixation de fractures des os.
